(19) Europäisches Patentamt
European Patent Office
Office européen des brevets

(11) **EP 2 870 472 B1**

(12) **EUROPEAN PATENT SPECIFICATION**

(45) Date of publication and mention
of the grant of the patent:
**26.09.2018 Bulletin 2018/39**

(21) Application number: **13732995.9**

(22) Date of filing: **04.07.2013**

(51) Int Cl.:
***G01N 33/18*** *(2006.01)*   ***C02F 1/42*** *(2006.01)*

(86) International application number:
**PCT/EP2013/064110**

(87) International publication number:
**WO 2014/006128 (09.01.2014 Gazette 2014/02)**

(54) **METHOD AND APPARATUS FOR DETERMINING THE TEMPORARY HARDNESS OF WATER**

VERFAHREN UND VORRICHTUNG ZUR BESTIMMUNG DER TEMPORÄREN WASSERHÄRTE

PROCÉDÉ ET DISPOSITIF POUR LA DÉTERMINATION DE LA DURETÉ TEMPORAIRE DE L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2012 EP 12175154**

(43) Date of publication of application:
**13.05.2015 Bulletin 2015/20**

(73) Proprietor: **Brita GmbH**
**65232 Taunusstein (DE)**

(72) Inventors:
• **WALDE, Hilmar**
**65510 Hünstetten-Wallbach (DE)**
• **NAGEL, Thomas**
**35796 Weinbach (DE)**
• **WEIDNER, Peter**
**92444 Rötz (DE)**
• **CONRADT, Berthold**
**65205 Wiesbaden (DE)**

(74) Representative: **van Lookeren Campagne, Constantijn August**
**Meissner Bolte Patentanwälte**
**Rechtsanwälte Partnerschaft mbB**
**Bankgasse 3**
**90402 Nürnberg (DE)**

(56) References cited:
**EP-A1- 2 169 392   DE-A1-102010 061 179**
**GB-A- 698 190**

• **Vernon L. Snoeyink, David Jenkins: "Water Chemistry", 1 January 1980 (1980-01-01), John Wiley & Sons**
• **"The Wordsworth Dictionary of Science & Technology", 1 January 1995 (1995-01-01), Wordsworth Editions Ltd., Ware, Hertf., England**
• **Judy Pearsall and Bill Trumble, ed.: "The Oxford English Reference Dictionary, 2nd Ed.", 1 January 1996 (1996-01-01), Oxford University Press**
• **"Function (mathematics)", , 8 September 2017 (2017-09-08), Retrieved from the Internet: URL:www.wikipedia.org [retrieved on 2017-09-08]**
• **"Function", , 8 September 2017 (2017-09-08), Retrieved from the Internet: URL:mathworld.wolfram.com [retrieved on 2017-09-08]**

**Description**

The invention relates to a method of determining a measure of hardness or temporary hardness.

The invention also relates to a system for determining a measure of hardness or temporary hardness.

The invention also relates to a system for treating water.

The invention also relates to a computer program.

EP 2 169 392 A1 discloses a method and apparatus for measuring the hardness of water. In one embodiment, the water to be measured is divided into several sub-streams in a distribution station, and directed to different treatment devices. After treatment of the sub-streams, these are directed in turn to a conductivity measurement device by valves in a second distribution station. The value concerned is then output to an evaluation device, in which the hardness of the water is determined. The advantage of this variant of the method is that only one conductivity measurement device is required. Due to this, offset errors are eliminated, which, in particular, makes possible a markedly more accurate determination of conductivity differences. It is further disclosed that one of the fractions of the water may be left untreated. The method is particularly suited for use with water blending devices. This means that blending of softened water with hard water is carried out beforehand. The hardness of the water after blending can be determined using the disclosed method.

A problem of the known method is that it requires multiple treatment devices, namely at least one for actual softening of the water and at least one for the subsequent measurement.

DE 10 2010 061 179 A1 discloses a method for generating drinking water with a selected hardness by means of an ion exchanger und a blending fixture for mixing drinking water treated in the ion exchanger with untreated drinking water from the water supply. The method includes the steps of:

(a) determining the hardness of the untreated drinking water by means of titration or another process with a high degree of accuracy;

(b) determining the conductivity of the untreated water with a conductivity sensor;

(c) conducting the entire flow of drinking water through the ion exchanger and determining the conductivity of the treated drinking water with a conductivity sensor;

(d) generating a calibration curve from the measurement values of the hardness and the conductivities determined in this way, on the assumption that the water hardness of the treated drinking water is smaller than $1°$ dH, preferably zero; and

(e) setting the blending proportion necessary for the selected hardness through the blending fixture by controlling to a target value of the conductivity corresponding to the selected hardness at the outlet of the blending fixture.

It is an object of the invention to provide a method, systems and computer program of the types defined above that can be used to obtain a relatively accurate measure of the hardness or temporary hardness, based on measurements of a property that depends also on the concentration of further components, and suitable for combination with the liquid treatment apparatus in a relatively efficient way.

This object is achieved according to one aspect by the method according the invention, which is defined in claim 1.

The term component is used herein to denote components suspended or dissolved in water, in particular relating to certain minerals, certain ion species or a definable proportion of certain ion species contributing to hardness or temporary hardness. The reference value may be a constant, in particular zero, or a known value of a variable.

The liquid treatment apparatus includes an inlet for untreated water, a branch point between the inlet and at least one first liquid path and at least one second liquid path and a mixing location, where the first and second liquid paths join. The mixing location is thus downstream of the treatment parts in the first liquid paths. Assuming the second liquid paths bypass all such treatment parts (but not necessarily treatment parts of a different type), the liquid treatment apparatus can be used to provide water that is a blend of water that has undergone the type of treatment that the treatment parts are configured to carry out and water that has not undergone such treatment. The proportion of the water that has not undergone such treatment is referred to as the blending fraction.

The liquid treatment part is generally configured to remove substantially all components of a certain type from the water that is led through it, for at least as long as it has not approached the end of its usable life. It may, however, be configured to remove these components to a known or knowable certain extent that is less than 100 %. To obtain a partial removal of these components from untreated water provided at the inlet, some of it is led through the second liquid path. The methods outlined herein are used to determine a value representative of the hardness or temporary hardness of water in either the untreated state or in the state prevailing downstream of the mixing location.

The methods outlined herein use measured values representative of values of a parameter of the water depending only partly on the concentration of components removable by the treatment part, but also on the concentration of other components. An example of such a parameter is the electrical conductivity, which depends on the total concentration of dissolved ion species, not just those causing temporary hardness. The ratio of other ion species to those causing temporary hardness is not fixed and generally not known exactly. By taking a difference between a first and second measurement value at different ratios of treated and untreated water, it is possible to separate out the contribution by components not removable by the liquid treatment part. It will be appreciated, for example, that subtracting a measured value obtained in a measurement carried out on fully treated water from a measured value obtained in a measurement carried out on fully untreated water could be converted into a measure of the concentration of components removable by the liquid treatment part in the untreated water. Thus, for example, using a conversion factor to convert conductivity to hardness, this subtraction would yield the temporary hardness of the untreated water if the treatment part were to be configured to remove temporary hardness completely.

In a typical water treatment apparatus, it is possible to carry out a measurement on the untreated water or on the water downstream of the mixing location, but it is difficult to carry out a measurement on the water as it flows between the treatment part and the mixing location, that is to say on the fully treated water. This section of the first liquid path is generally contained in either a replaceable cartridge containing the liquid treatment part or in a filter head to which such a cartridge is connectable. The methods outlined herein use a first measurement value obtained from a first measurement made downstream of the mixing location and a second measurement value that is either also obtained from a measurement made downstream of the mixing location or from one carried out on the untreated water. Thus, this access problem does not occur, and it is possible to use the liquid treatment apparatus actually being used to treat the water also in the determination of the measure of the concentration of components removable by the liquid treatment part of the liquid treatment apparatus, instead of a separate device located upstream of the actual liquid treatment apparatus as proposed in EP 2 169 392 A1.

In case the measurement to obtain the second measurement value were to be a measurement carried out downstream of the mixing location at a value of the blending fraction of essentially zero, then it would be possible to determine an output value pertaining to the blended water (i.e. the water downstream of the mixing location), but not one pertaining to the untreated water, using essentially only the first and second measurement values (and optionally one or more conversion relations to arrive at a measure having a different dimension than that of the measurement values). The methods outlined herein involve determining the output value as a function of at least a value representative of the blending fraction prevailing at the first measurement relative to a reference value, which may be zero. This value of the blending fraction is either known (or assumed known) because the blending fraction is set to this value, or it can be measured. Because of this further input, it is possible to obtain a relatively accurate measure of the concentration of interest in the untreated water using one or two measurement values obtained from a measurement downstream of the mixing location. This would not be possible merely by taking the difference between the first measurement value and a second measurement value obtained from a measurement carried out on the untreated water, for example. This is because the measurement values are representative of respective values of a parameter of the water depending partly and not only on the concentration of components removable by the treatment part.

It is observed that, for the present purposes, a value corresponding to the fraction of the untreated water led through the at least one first path is also a value representative of the blending fraction, since all of the water entering through the inlet is led through either a first or a second path. It is merely convention to refer to the blending fraction.

In an embodiment, the second measurement value is obtained from a measurement downstream of the mixing location at a different value of the blending fraction than the first.

An effect is that the measurement values can be obtained from the same sensor (at successive points in time). The measurements are carried out on at least partially treated water. Typically, the liquid treatment part will remove components that also impact on the operation of the sensor. By using a sensor arranged to carry out measurements on at least partially treated water, the lifetime of the sensor can be increased. Because the measurement values can all derive from measurements made using the same sensor, the problem of sensor drift becomes less acute. It will be recalled that the output value is determined as a function of the difference between the two measurement values. Where a single sensor is used, systematic errors due to sensor drift are thus eliminated, the successive measurements occurring in much closer succession than the time scale on which sensor drift occurs to any meaningful extent. Were use to be made of two sensors, then different and independently varying systematic errors due to sensor drift would affect the output value. Such an error would be all the more likely to occur where one sensor is permanently exposed to untreated water and the other is not. The only remedy would be relatively frequent re-calibrations, the need for which is thus reduced or eliminated in this embodiment.

In a variant of this embodiment, the liquid treatment apparatus includes at least one device for adjusting the blending fraction, and the first and second measurement values are obtained by causing the device to adjust the blending fraction.

This variant ensures that the first and second measurement values are obtained from measurements at different values of the blending fraction. The device for adjusting the blending fraction will generally include a motor, for example a servomotor or stepper motor, coupled to one or more valves. It may further include a torque-converting gear unit.

A variant includes determining at least one further output value, each determined as a function of at least a difference between a further measurement value obtained from a further measurement made downstream of the mixing location and the second measurement value.

In particular where large variations in the blending fraction are used to obtain measurement values at different values of the blending fraction, this embodiment removes the need for frequent re-adjustment to such blending fraction values deviating significantly from a desired value. This protects appliances provided with the mix of treated and untreated water and/or removes the need for disconnecting such appliances whilst the measurements at such deviating blending fraction values are carried out.

In an alternative embodiment, the reference value corresponds to a second value of the blending fraction, prevailing at the measurement used to obtain the second measurement value so that the output value is determined as a function of a difference value representative of a change in the blending fraction, and the step of determining the output value includes dividing the difference between the first and second measurement value by the difference value.

An effect is to avoid large variations in the blending fraction, such as are caused by setting the blending fraction to zero, altogether. In effect, an approximation of the derivative of the parameter value in the blended water with respect to the blending fraction is determined. This derivative corresponds to the difference in the parameter value immediately upstream and immediately downstream of the liquid treatment part. In other words, it corresponds to the change in parameter value due to the removal of certain components by the liquid treatment part. Consequently, the difference value can be converted directly into a measure of the concentration of the components removable by the liquid treatment part in the untreated water.

As an example, assuming the parameter is the electrical conductivity s of water, and the components of interest are those removable by a water softener. Let the electrical conductivity of the untreated water be $s_0$ and the electrical conductivity of the water immediately downstream of the liquid treatment part (i.e. of the fully treated water before mixing) be $s_1$. Let the blending faction be $x$ and the electrical conductivity downstream of the mixing location be $s(x)$. Then the following relation pertains:

$$s(x) = x \cdot s_0 + (1-x) \cdot s_1 = (s_0 - s_1) \cdot x + s_1 = \Delta s \cdot x + s_1 \; . \qquad (1)$$

The change in electrical conductivity due to the removal of the hardness equals $\Delta s$. This value is obtainable by taking the derivative $s'(x)$, as will be apparent from equation (1). An approximation of the derivative at a particular set value $x^*$ of the blending fraction is:

$$s'(x^*) = \frac{s\left(x^* + \frac{\Delta x}{2}\right) - s\left(x^* - \frac{\Delta x}{2}\right)}{\Delta x} \; . \qquad (2)$$

It follows that a small deviation either way from the set value x* of the blending fraction suffices to obtain the difference value $\Delta s$.

Because this variant does not require the blending fraction to be set to zero, there is no need to disconnect vulnerable apparatus downstream of the mixing location to protect it. For example, where the liquid treatment part is arranged to remove temporary hardness from water, reducing the blending fraction too much would make the water downstream of the mixing location quite acid, increasing the likelihood of corrosion. This can be avoided in this variant, since it can be carried out by varying the blending fraction only by a small amount around a value suitable for supplying essentially neutral water.

In an embodiment, wherein the apparatus includes at least one device for adjusting the blending fraction, the method includes causing the device to adjust the blending fraction in dependence on the output value.

This embodiment results in a liquid treatment apparatus with an automatically controllable blending fraction. It is able to provide water with a desired degree of total hardness or a desired degree of temporary hardness, depending on the constitution of the liquid treatment part. In the process, the acidity level of the water is also set to a value appropriate to avoid corrosion in devices downstream of the mixing location, without the need to use a buffering agent in the liquid treatment part.

In an embodiment, the measurement values are representative of electrical conductivity of water.

Thus, a direct electrical signal is provided, so that transducers to convert a different physical variable into an electrical signal for processing by a signal processing unit are dispensed with. The electrical conductivity of a liquid is directly related to the concentration of dissolved ions. As such, it is not suitable for directly determining the concentration of only a sub-set of all ion species but a measure of this concentration can be achieved with this method and the appropriate choice of liquid treatment part.

In an embodiment, the measure is a measure of temporary hardness.

The method is particularly suitable for this measure. Hardness in liquid is due to magnesium and calcium ions.

It comprises two components, namely temporary hardness and permanent hardness. Temporary hardness is caused by dissolved minerals with carbonate and bicarbonate anions, whereas permanent hardness is associated with minerals with other anions, such as chloride. Even if one were to use ion-selective conductivity sensors responsive to calcium and/or magnesium, it would be impossible to distinguish between the cations associated with temporary hardness and those associated with permanent hardness. By using a liquid treatment part effective to remove only temporary hardness, the method is able to determine the temporary hardness of the untreated liquid and/or of the mix of untreated and treated liquid provided by the liquid treatment apparatus. Compared to methods that use tables to relate total hardness or electrical conductivity to temporary hardness or those that use one-time measurements (titrimetry for instance), the methods outlined herein are relatively accurate in the face of varying concentrations of other minerals in the untreated liquid.

In a particular variant, the liquid treatment part is a liquid treatment part comprising a weakly acidic ion exchange medium through which the liquid is led.

In a particular variant, the liquid treatment part is a liquid treatment part comprising an ion exchange medium that is at least initially in the hydrogen form. This results in a relatively strong signal, because cations are removed from the liquid in exchange for $H^+$ ions, which react to water and carbon dioxide, so removing cations from the liquids. The result is a relatively marked change in electrical conductivity, in particular. In the alternative, there would still be a change, due to differing activity levels of the cations removed and those released in exchange, but this change would be smaller. The ion exchange medium in the hydrogen form may be the only type of cation exchange medium used to treat the liquid, such that liquid at the mixing location has been exposed in part to ion exchange medium only in the hydrogen form and in part to no cation exchange medium or no ion exchange medium at all.

In an embodiment, the measurement values are further dependent on temperature.

In one variant, a sensor is used to determine a temperature value and data representative of measured values of the electrical conductivity of the water are processed to correct these measured values in dependence on the temperature value. In another variant, a sensor incorporating, for example, a temperature-dependent resistor, is used to obtain a measured value dependent on both temperature and the value of the electrical conductivity of the water. In either case, the electrical conductivity value is in effect normalised to a value that would pertain at a reference temperature.

The electrical conductivity and pH of a liquid are both dependent on ion concentration and ion activity, the latter being temperature-dependent.

In an embodiment, the liquid treatment part is included in a replaceable cartridge for coupling to a device including at least the inlet of the liquid treatment apparatus.

The methods outlined herein are particularly adapted for use in such an embodiment, because they do not require measurements to be made immediately downstream of the liquid treatment part, i.e. upstream of the mixing location in the first liquid path. This stretch of the first liquid path is generally located within the cartridge, and it would be cumbersome to have to mount sensors there. Instead, the methods outlined herein can function with a sensor located in the device to which the removable cartridge is coupled, or further downstream of that device.

In a variant, the method includes obtaining input data identifying at least a type of the replaceable cartridge, and at least one of executing and adapting the method in dependence on the identified type.

The method can be executed in dependence on the identified type in the sense that it is only executed if the type is one of at least one pre-determined type. This ensures that the method is only executed if the cartridge contains an appropriate fluid treatment medium. The variant is also suitable for use with removable cartridges containing treatment parts that remove different types of components, particularly ion species, to a different extent. The input data identifying the type of cartridge can be related to stored data representative of the types of components removed and the effectiveness of the liquid treatment part in removing the types of components concerned.

Thus, in a particular variant, the output value is determined as a function of at least one value associated with the type of the replaceable cartridge, in particular the extent to which at least some types of components are removed from water led through the bypassed liquid treatment part.

An embodiment of the method includes:

> obtaining a measure of an amount of water led through at least one first liquid path within a certain time period; and using at least this measure and a measure of the hardness or temporary hardness of the water determined for the certain time period in a determination of a measure of a state of exhaustion of the liquid treatment part in the first liquid path.

In this embodiment, an integral of the flow rate, weighted according to the concentration of components removable by the liquid treatment part, is determined. This represents the accumulated load on the liquid treatment part during its lifetime. It allows an automated system carrying out this embodiment to determine the state of exhaustion, or conversely the remaining useful lifetime, of the liquid treatment part and/or of the fluid treatment medium it contains. Where the liquid treatment part is comprised in a replaceable cartridge, the system carrying out this embodiment is able to determine when the cartridge should be replaced. In one variant, an output representative of the state of exhaustion is caused to be provided by an output device in a form perceptible to a user. In other words, an audible or visible signal is provided to the user. Additionally or alternatively, output data representative of the state of exhaustion are communicated to an external device, for example an appliance arranged to receive the treatedwater, via a data communication link. This would allow the user interface of the external device to be used to provide a signal to the user. It is useful where the liquid treatment apparatus is located out of sight of the user but the appliance is not. In a variant, the output perceptible to a user is only provided upon determining that water is being drawn from the liquid treatment apparatus. This increases the likelihood that a user is actually present to observe the output, and saves energy. Similarly, the communication of output data to an external device via a communication link can be effected in dependence upon a determination that water is being drawn from the liquid treatment apparatus. This increases the likelihood that the data will be processed, since the external device is unlikely to be switched off or in a standby mode.

According to a further aspect, a system for determining a measure of hardness or temporary hardness according to claim 8 is provided.

The system can be integrated with the liquid treatment apparatus, because use is made of the liquid treatment part that is part of the liquid treatment apparatus. It is thus cheaper and simpler than a separate system situated downstream or upstream of a liquid treatment apparatus with a liquid treatment part and an adjustable bypass. The system still delivers an output value that is suitable for controlling the blending fraction of the liquid treatment apparatus. It is able to determine the concentration of components removable from the untreated water by the liquid treatment part in the untreated water or in the mix of treated and untreated water provided by the liquid treatment apparatus. The measured parameter that allows the system to do so need not itself be representative of only the components removable by the liquid treatment part, but may depend also on the concentration of further components that remain in the water. The system requires no sensors between the liquid treatment part and the mixing location. This makes it possible to implement the system by adapting only the operating methods of existing apparatus, rather than the hardware between the branch point and the mixing location.

An embodiment of the system is configured to obtain the second measurement value from a measurement downstream of the mixing location at a different value of the blending fraction than the first.

This embodiment makes use of only one sensor, reducing the costs of implementing it and removing a source of error due to the use of different sensors with different and differently varying systematic errors.

In an embodiment, wherein the liquid treatment apparatus includes at least one device for adjusting the blending fraction, the system includes a component for causing the device to adjust the blending fraction to obtain the first and second measurement values.

This embodiment is an implementation of a system that can function with only one sensor. The device for adjusting the blending fraction will generally include an electric motor, for example a servomotor or stepper motor. It may further include a torque-converting gear unit.

An embodiment of the system is configured to determine at least one further output value, each determined as a function of at least a difference between a further measurement value obtained from a further measurement made downstream of the mixing location and the second measurement value.

This embodiment is configured to interrupt the normal operation of the liquid treatment apparatus relatively infrequently. It is especially of use where the second measurement value is obtained at a blending fraction deviating significantly from an optimum for the water to be delivered by the liquid treatment apparatus.

An embodiment of the system is configured to use a reference value corresponding to a second value of the blending fraction, prevailing at the measurement used to obtain the second measurement value so that the output value is determined as a function of a difference value representative of a change in the blending fraction, and to divide the difference between the first and second measurement value by the difference value as part of the determination of the output value.

This embodiment can be implemented in such a way that the blending fraction deviates only slightly from a set value.

An embodiment of the system is configured to cause a device for adjusting the blending fraction to adjust the blending fraction in dependence on the output value.

This embodiment constitutes a system for controlling the concentration of components removable by the liquid treatment part of the liquid treatment device in fluid delivered by the liquid treatment apparatus.

An embodiment of the system further includes the at least one sensor, wherein the sensor is arranged to provide measurement values representative of electrical conductivity of the water.

The electrical conductivity is in particular dependent on the ion concentration. Where the liquid treatment part is configured to remove ions of some species only or only some salts, the system is nevertheless able to determine a measure of the concentration of such components relatively accurately.

In an embodiment, the system is configured to determine the measure in the form of a measure of temporary hardness.

An embodiment further includes a device for obtaining measurement values that are each dependent on both temperature and a respective value of the electrical conductivity of the water.

This embodiment is useful to compensate for any effects the temperature may have on the concentration-dependency of the measured electrical conductivity.

In a variant, the system further includes at least one sensor for sensing values of the electrical conductivity of the water, which sensor is arranged in a common housing with a temperature sensor.

The temperature value thus corresponds more closely to the temperature of the water on which the electrical conductivity measurement is carried out.

An embodiment of the system further includes a device including at least the inlet of the liquid treatment apparatus and adapted for coupling to a replaceable cartridge including at least the bypassed liquid treatment part of the liquid treatment apparatus.

This embodiment takes account of the fact that different liquid treatment parts may be required for different types of untreated water, and that some types of exhaustable fluid treatment medium cannot easily be regenerated on site.

In a variant, the system includes an interface for obtaining input data identifying at least a type of the removable cartridge, and it is configured to carry out the determination of the output value in dependence on the identified type.

This variant is thus suitable for use with different types of cartridge, not just a particular prescribed one.

A variant of the system is configured to determine the output value as a function of at least one value associated with the type of the replaceable cartridge.

This variant is, for example, suitable for use with cartridges with an internal bypass.

An embodiment of the system, adapted for use with a device including at least the inlet of the liquid treatment apparatus and adapted for coupling to a replaceable cartridge including at least the bypassed liquid treatment part of the liquid treatment apparatus, further includes a housing in which at least one of the at least one sensors is arranged, which housing is provided with:

a liquid inlet provided with a coupling device for coupling to an outlet of the device including at least the inlet of the liquid treatment apparatus; and
a liquid outlet provided with a coupling device for coupling to a liquid conduit.

This variant is suitable for retrofitting existing liquid treatment apparatus with adjustable bypass, since the housing can be connected to the outlet of such an existing liquid treatment apparatus.

According to another aspect, a system for treating water according to claim 13 is provided.

In an embodiment of the system, the liquid treatment part is configured to remove temporary hardness to at least a certain extent.

According to another aspect of the invention, there is provided a computer program including a set of instructions capable, when incorporated in a machine-readable medium of causing a system having information processing capabilities, a liquid treatment apparatus for use in a method according to the invention and an interface for obtaining measurement values representative of respective values of the electrical conductivity of water
to carry out a method according to the invention.

The invention will be explained in further detail with reference to the accompanying drawings, in which:

Fig. 1 is a schematic diagram of a water treatment apparatus including a system for measuring and controlling temporary hardness;
Fig. 2 is a flow chart illustrating a method of determining a measure of the temporary hardness;
Fig. 3 is a flow chart illustrating an alternative method of determining a measure of the temporary hardness;
Fig. 4 is a schematic diagram of a variant of the water treatment apparatus of Fig. 1 including a fluid treatment device in the form of a replaceable cartridge; and
Fig. 5 is a very schematic diagram of an alternative water treatment apparatus including a system for measuring and controlling temporary hardness.

In the following, the example of a liquid treatment system for softening water will be used. The apparatus is equally suitable for treating other types of liquid, and the methods discussed below for determining a measure of hardness can also be used to determine the concentration of components removable by a liquid treatment part of a liquid treatment device for treating such other liquids.

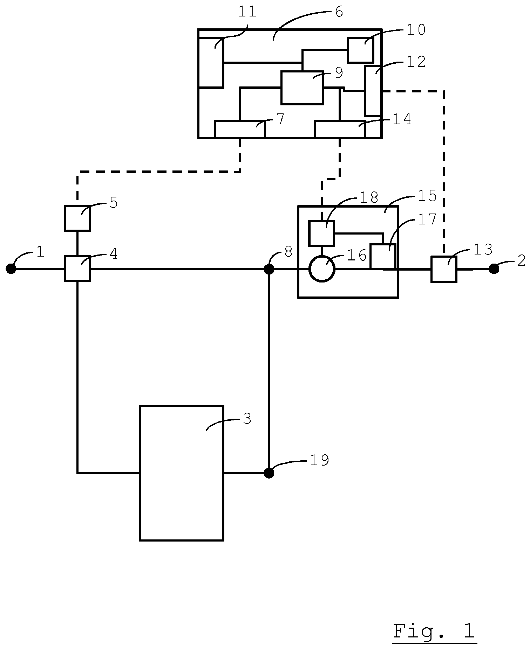

A water treatment apparatus illustrated by way of example in Fig. 1 includes an inlet 1 for receiving untreated water and an outlet 2 for providing water with a desired level of temporary hardness. The inlet 1 is connectable to a supply of untreated water, in particular the mains water supply. The outlet 2 is connectable to an appliance such as a steam cooker, dish washer or a device for preparing beverages (e.g. a coffee maker). The fluid treatment apparatus includes a fluid treatment device 3 configured to remove temporary hardness to at least a certain extent from the water led through it. In the following, it will be assumed for simplicity that the fluid treatment device 3 is effective to remove essentially all temporary hardness. Temporary hardness, also known as carbonate hardness, is caused by the presence of dissolved carbonate minerals (essentially calcium carbonate and magnesium carbonate). It is distinguished from permanent hardness, to which other minerals such as calcium chloride also contribute.

In an alternative embodiment, the fluid treatment device 3 is effective to remove not just the temporary hardness, but all hardness from the water led through it, and the fluid treatment apparatus is configured to supply water with a desired level of total hardness. This is merely a question of using a fluid treatment device 3 with a different filter medium. Returning to the example of temporary hardness, the fluid treatment device 3 can comprise a filter medium including a weakly acidic ion exchanger that is at least initially in the hydrogen form, for example.

There is thus a first fluid path running from the inlet 1 through the fluid treatment device 3 to the outlet. There is also a second fluid path from the inlet 1 to the outlet 2. This second fluid path bypasses the fluid treatment device 3. In the illustrated embodiment, the water passing through the second fluid path is not treated at all, to simplify the discussion.

A variable ratio flow divider 4 is arranged at a branch point where the first and second fluid paths go their separate ways. The flow divider 4 is adjustable by a motor 5 controlled by a control device 6 provided with an interface 7 to the motor 5. In this way, an adjustable fraction of the water passing through the inlet 1, which fraction is referred to herein as a blending fraction $x$, is led through the second fluid path. The first and second fluid paths join at a mixing location 8, so that the water treated by the fluid treatment device 3 and that left untreated can mix. In the illustrated embodiment, the control device 6 is programmed to relate positions of the motor 5 to values of the blending fraction $x$ and vice versa.

The control device 6 includes a data processing unit 9 and memory 10. It is provided with an interface 11, e.g. a user interface for receiving input relating to a target value of the temporary hardness of the water at the outlet 2. The input may be a value or information representative of the type of application the water is to be used in. In an alternative embodiment, such input can be communicated from another device through the interface 11. The control device 6 is configured to determine the temporary hardness of the water at the inlet and to set the blending fraction $x$ to the appropriate value by adjusting the settings of the motor 5 and flow divider 4. In this way, a particular target value of the temporary hardness can be achieved even though the fluid treatment device 3 is able to remove the temporary hardness from water led through it to only a fixed extent, generally 100 %.

In the illustrated embodiment, the control device 6 includes an interface 12 to a flow meter 13 configured to measure the volumetric flow through the fluid treatment apparatus. Since the control device 6 controls the settings determining the blending fraction $x$, it can convert the total accumulated volumetric flow through the fluid treatment apparatus into a value representative of the volume of water that has passed through the fluid treatment device 3. Since it also determines the temporary hardness of the water at the inlet 1, it is able to determine a measure of the total amount of temporary hardness-inducing components to which the fluid treatment device 3 has been exposed over a certain period (generally commencing at first use). In this way, it can signal when the fluid treatment device has become exhausted and is to be replaced or regenerated. This signal is provided through the interface 11. Where the interface 11 is a user interface, the signal is provided in a form perceptible by a user, e.g. by way of a visible and/or audible indication of the remaining lifetime of the fluid treatment device and/or of the percentage of initial capacity that has been used up. Where the interface 11 is a data communication interface for establishing a data communication link to an external appliance, the same information is communicated in the form of a data signal for processing and/or output by the external appliance. Optionally, the signal is provided only upon determining that fluid is being provided from the fluid treatment apparatus.

The control device 6 is also provided with an interface 14 to a sensor device 15 including an electrical conductivity sensor 16 arranged to measure the electrical conductivity of the water downstream of the mixing location 8. The electrical conductivity of water is dependent on the concentration of dissolved ions of all species, not just those contributing to hardness or that fraction of those species contributing to hardness that contributes to the temporary hardness. Thus, for example, the water can contain dissolved calcium chloride, of which the calcium ions do not contribute to temporary hardness, but do contribute to permanent hardness. Moreover, certain ion species do not contribute to hardness at all, but their concentration partly determines the electrical conductivity of the water. The control device 6, specifically the data processing unit 9, is programmed to determine the temporary hardness of the untreated water using measurement values obtained from the sensor device 15.

In the illustrated embodiment, the sensor device 15 includes a temperature sensor 17 and a data processor 18 for converting electrical conductivity values from the conductivity sensor 16 into values that would have been obtained if the temperature had been at a certain reference value, e.g. 25°C. These corrected values are provided as output to the control device 6. This takes account of the fact that the electrical conductivity for a given concentration varies with the temperature of the water. The temperature signal need not be provided to the control device 6, saving on connectors and leads and reducing the potential for failures.

The electrical conductivity of the water at the location of the sensor device 15, i.e. downstream of the mixing location 8 is dependent on the electrical conductivity of the untreated water, the blending fraction and the electrical conductivity of the water at an exit point 19 directly downstream of the fluid treatment device 3, but upstream of the mixing location 8. Let the electrical conductivity of the untreated water be $s_0$ and the electrical conductivity at the exit point 19 be $s_1$. The difference $\Delta s \equiv s_0\text{-}s_1$ is due to the removal of the temporary hardness, i.e. representative of a change in the electrical conductivity due to treatment by the fluid treatment device 3. This value is to be obtained and converted into a measure of temporary hardness. The electrical conductivity of the water at the location of the sensor device 15 is given by equation (1), repeated here for ease of reference:

$$s(x) = x \cdot s_0 + (1-x) \cdot s_1 = (s_0 - s_1) \cdot x + s_1 = \Delta s \cdot x + s_1 \, . \tag{1}$$

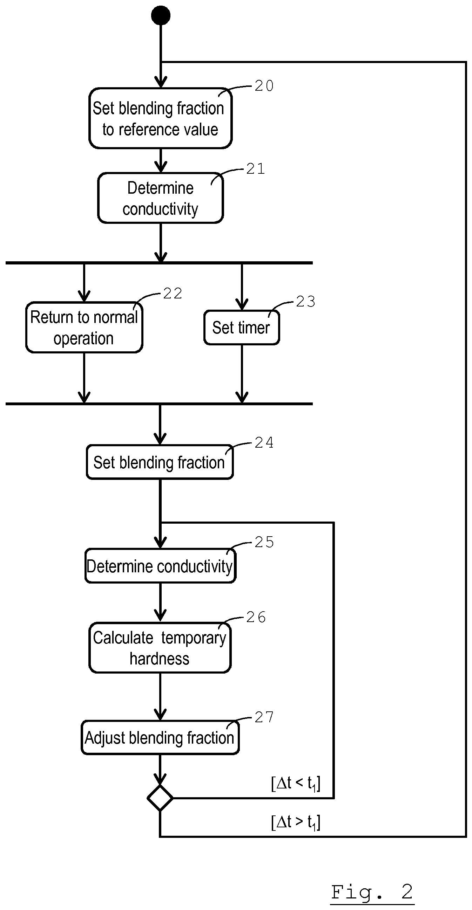

A method of determining the temporary hardness of the untreated water and a method of determining the temporary hardness of the treated water will now be described with reference to Fig. 2.

In a first step 20, the control device 6 sets the blending fraction $x$ to a reference value $x_1$ by causing the motor 5 to adjust the variable ratio flow divider 4. In particular, the reference value $x_1$ is set to zero, corresponding to a completely closed second fluid path.

The associated value of the electrical conductivity $s(x_1)$, which value is already corrected for any deviations from a reference temperature, is then obtained (step 21). This value is stored in memory 10. The control device then returns to normal operation (step 22). It also sets a timer (step 23) to enable it to repeat the first step 20 at an appropriate later point in time.

The blending fraction is then set (step 24) to an appropriate second value $x_2$, different from the reference value $x_1$. This value $x_2$ can be a default value determined by the target temporary hardness and the latest available value for the temporary hardness of the untreated water (or a default value in case the control device 6 is being used for the very first time). The control device 6 then again obtains (step 25) from the sensor device 15 a measurement value representative of the electrical conductivity and corrected for deviations from a reference temperature. This step 25 is executed after a delay that is long enough to ensure that the sensor device 15 measures the electrical conductivity at the new value $x_2$ of the blending fraction.

In case the control device is configured to determine a measure of the temporary hardness $H$ of the untreated water it proceeds as follows:

$$H = \frac{s(x_2) - s(x_1)}{(x_2 - x_1) \cdot F} \, , \tag{3}$$

where F is a conversion factor. The conversion factor $F$ is a constant in one embodiment. It can be equal to approximately 30 $\mu$S/°dH, where dH stands for deutsche Harte.

Where the reference value of the blending fraction $x_1$ is zero, the determination is simplified:

$$H = \frac{s(x_2) - s(x_1)}{x_2 \cdot F} \, . \tag{4}$$

In embodiments in which the temporary hardness $H^*$ of the blended water is determined in this step 26, the calculation becomes:

$$H^* = \frac{x_2 \cdot (s(x_2) - s(x_1))}{(x_2 - x_1) \cdot F} \, . \tag{5}$$

Where the reference value of the blending fraction $x_1$ is zero, the determination is simplified further:

$$H^* = \frac{(s(x_2) - s(x_1))}{F} \, . \tag{6}$$

Thus, where the value $s(x_1)$ of the electrical conductivity is a value obtained from a measurement downstream of the mixing location 8 at a reference value $x_1$ of the blending fraction of exactly zero, it is sufficient to determine the difference between the further value $s(x_2)$ of the electrical conductivity and the value $s(x_1)$ obtained at the reference value $x_1$ of the blending fraction. Knowledge of the exact value of the blending fraction is not required.

With knowledge of the temporary hardness $H$ of the untreated water or the temporary hardness $H^*$ of the water downstream of the mixing location 8, the control device 6 proceeds (step 27) to adjust the blending fraction $x$ to a new value $x_3$ in accordance with a target value, if required. The target value is a value appropriate to the type of appliance connected to the outlet 2.

At a later point in time, the steps 25-27 of determining the conductivity, calculating the temporary hardness and adjusting the blending fraction $x$ if required are repeated. However, the value $s(x_1)$ of the electrical conductivity at the reference value $x_1$ that is used in the calculations remains the same one as used in the previous iteration. In particular, the blending fraction is not re-set to zero first. In one embodiment, these steps 25-27 are repeated at pre-set intervals. The interval can be in the order of hours, a day or even more. Instead of using pre-set time intervals, the passing of a pre-determined volume of water through the system or a change in conductivity exceeding a pre-set level can trigger a repeat of these steps 25-27.

Returning to the illustrated example, once more than a pre-determined interval of time $t_1$ has elapsed since the timer was set (step 23), the control device 6 proceeds to obtain a new reference value of the electrical conductivity $s(x)$ by returning to the first step 20, in which the blending fraction $x$ is set to a reference value, in particular zero. Instead of using a pre-determined interval of time $t_1$, it is also conceivable to return to the first step 20 after a pre-determined number of iterations of the steps 25,26,27 using the same value $s(x_1)$ of the electrical conductivity at the reference value $x_1$ of the blending fraction. In yet another embodiment, re-use of the same reference value $s(x_1)$ of the electrical conductivity ceases once a determined output value deviates from a preceding value by more than a certain amount. It is noted that, if the same value $s(x_1)$ is used for too long, changes in electrical conductivity due to variations in the concentration of other components than hardness-inducing components could lead to errors in the determined measure of temporary hardness.

In an embodiment, this repeat execution of the first step 20 is preceded by an output signal, for example provided via the user interface 11, so that any appliance connected to the outlet 2 can be disconnected first. In another embodiment, a flow diverter (not shown) is connected to the outlet 2, controlled by the control device 6 and connected to both the water-consuming appliance and a drain, so that the control device 6 can cause the water that flows out of the outlet 2 when the blending fraction $x$ is set to zero to be discharged down the drain. Alternatively, it is possible to use a fluid treatment device containing a buffering agent. This ensures that appliances connected to the outlet 2 do not receive water with a very low pH when the blending fraction $x$ is set to zero. However, where a buffering agent is used, a different value for the conversion factor $F$ may need to be used, because the change in electrical conductivity due to the fluid treatment will generally be smaller (ions are released into the fluid). Alternatively, a correction factor may be applied to the measured values of the electrical conductivity.

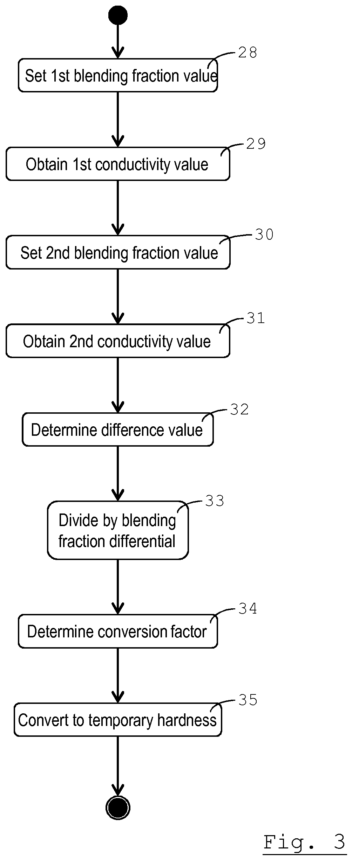

A different embodiment of a method of determining a measure of temporary hardness is illustrated in Fig. 3. it involves obtaining a value approximating the derivative of the electrical conductivity (corrected for deviations from a reference temperature) with respect to the blending fraction so as to determine the change in electrical conductivity caused by the treatment effected by the fluid treatment device 3.

It is assumed in the following that the blending fraction x is at a certain value $x_0$. At first use of the system this value $x_0$ can be a default value or a value dependent on a target value for the temporary hardness at the outlet 2 and a default value representative of a common level of temporary hardness.

In a first step 28, the control device 6 causes the motor 5 and flow divider 4 to adjust such that the blending fraction is changed to a first value $x_1$, e.g. $x_1 = x_0 + \Delta x/2$. In a next step 29, a measurement value $s(x_1)$ is obtained from the sensor device 15, the value pertaining to a measurement carried out with the blending fraction at the first value. Next (step 30), the control device 6 causes the motor 5 and flow divider 4 to adjust such that the blending fraction is changed to a second value $x_2$, e.g. $x_2 = x_0 - \Delta x/2$. Then (step 31), a second measurement value $s(x_2)$ is obtained from the sensor device 15, the value pertaining to a measurement carried out with the blending fraction at the second value $x_2$.

The control device can now determine (step 32) a difference value $s(x_2)-s(x_1)$ and (step 33) divide this value by the blending fraction differential $\Delta x$ to obtain an approximation of the derivative of the conductivity $s(x)$ with respect to the blending fraction $x$, which, it will be recalled, approximates the difference in conductivity caused by the temporary hardness that is removable by the fluid treatment device 3. It then obtains the value of a conversion factor F (step 34) and converts (step 35) the output of the preceding step 33 into a value of the temporary hardness. For example, the conversion can be carried out by dividing the result of the division step 33 by a constant conversion factor $F = 30\ \mu S/°dH$, where dH stands for deutsche Harte.

The change in blending fraction $\Delta x$ can be quite small, of the order of 0.2 or smaller, e.g. 0.1. As a consequence, any appliance connected to the outlet 2 can remain connected whilst the method of Fig. 3 is carried out. It can be repeated relatively often, but intervals of the order of one or more days generally suffice to capture variations in the temporary hardness supplied to the inlet 1.

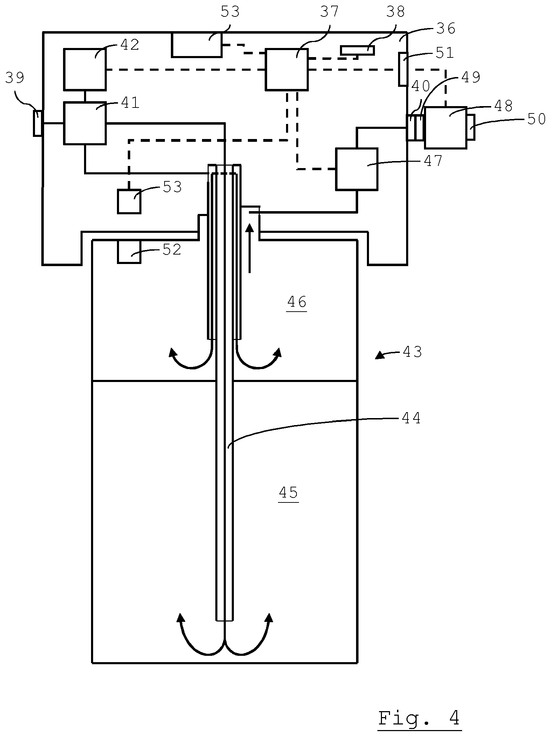

In Fig. 4, a variant of the water treatment system of Fig. 1 is shown in a very schematic manner. It includes a filter head 36 comprising a housing for a data processing unit 37 and memory 38. The housing is provided with an inlet connector 39 for coupling to a supply of untreated water. It further comprises an outlet connector 40 for connection to a conduit.

In the illustrated embodiment, the housing further includes a variable ratio flow divider 41 and a motor 42 for adjusting the variable ratio flow divider 41.

The filter head 36 is adapted for coupling to a replaceable cartridge 43. The filter head 36 is provided with two fluid outlets that are placed in fluid communication with respective inlets of the cartridge 43 in such a way that the connection is sealed from the environment. The filter head 36 is provided with one fluid inlet that is configured to be placed in fluid communication with an outlet of the cartridge 43 when the cartridge 43 is coupled to the filter head 36.

Water entering the cartridge 43 through a first of the inlets is conducted through a fall tube 44 to emerge at an end of a first bed 45 of fluid treatment medium forming a first fluid treatment part. This first bed 45 can, for example, include a treatment medium configured to remove temporary hardness from the water, e.g. an ion exchange resin or a chelating resin. In this example, it comprises a weakly acidic ion exchange resin (and no other type of ion exchange resin). The ion exchange resin is predominantly in the hydrogen form, at least on first use. The water passed through the first bed 45 is subsequently led through a second bed 46 including a treatment medium that is generally not configured to remove temporary hardness, although it may be configured to remove only a (known) fraction of the temporary hardness. As an example, the second bed 46 can comprise activated carbon, optionally impregnated with an oligodynamic substance such as silver.

Water entering the cartridge 43 through the second of the inlets bypasses the first bed 45. It is mixed with the water that has been led through the first bed 45 in the second bed 46 so that the mixing location is in the second bed 46 of the cartridge 43. The settings of the flow divider 41 determine the proportion of the water that flows only through the second bed 46, which proportion forms the blending fraction x.

The water that has mixed in the second bed 46 is led out of the cartridge 43 into the filter head 36. It passes through a flow meter 47. In the illustrated example, the flow meter 47 is located in the filter head 36, but it may be external to the filter head 36, being located either upstream or downstream thereof. The signal from the flow meter 47 is passed to the data processing unit 37. Because the data processing unit 9 also controls the settings of the motor 42 and flow divider 41, it is able to calculate the volume of water that has passed through the first filter bed 45. Because it further determines the temporary hardness of the untreated water, the data processing unit 37 is able to determine when the cartridge 43 is to be replaced.

The water leaves the filter head 36 through the outlet connector 40. A sensor device 48 of the type described above with reference to Fig. 1 is contained in a housing provided with an inlet connector 49 that is coupled to the outlet connector 40 of the filter head 36. The sensor device 48 is also provided with an outlet connector 50 of the same type as the outlet connector 40 of the filter head 36. Thus, it is connectable to an appliance arranged to use the water with the desired temporary hardness provided by the fluid treatment apparatus. The signal from the sensor device 48 is provided to the data processing unit 37 through an interface 51 of the filter head 36.

The cartridge 43 can be one of several different types. It is provided with a machine-readable token 52, e.g. a bar code, RFID tag or similar device. The filter head 36 is provided with a device 53 for at least obtaining information from the token.

The reader device 53 is arranged to provide a signal to the data processing unit 37. Thus, the data processing unit 37 is arranged to obtain input data identifying at least a type of the cartridge 43, type information being among the information stored in the token 52.

The data processing unit 37 is arranged to carry out one of the methods of Figs. 2 and 3. It is provided with an interface 54 for receiving information representative of a target value for the temporary hardness of the water to be provided by the fluid treatment apparatus, or information enabling it to derive the target value. The interface 54 may comprise a communication interface for exchanging data with a further device. It may also or alternatively comprise a user interface. In one embodiment, the data processing unit 37 is arranged to provide output data representative of the temporary hardness of at least one of the untreated water and the water provided through the outlet connector 40.

In an embodiment, the data processing unit 37 enables the filter head 36 to carry out a variant of one of the methods of Figs. 2 and 3 in which the temporary hardness is determined in dependence on the identified type of the cartridge 43. In particular, the data processing unit 37 first determines that the cartridge 43 is of a type suitable for carrying out a method as described above. Then, in an embodiment, the temporary hardness of the untreated or treated water is determined as a function of at least one value associated with the type of the cartridge 43, there being a different value associated with each of several possible types in the memory 38. In particular, the extent to which the medium in the first bed 45 is arranged to remove temporary hardness and/or the extent to which certain ion species not associated with temporary hardness are removed by the medium in the first bed 45 can be stored in association with each type of cartridge 43. Thus, the data processing unit 37 would use a different factor $F$ in equations (3)-(6) (corresponding to step 26 in the method of Fig. 2 and step 34 in the method of Fig. 3).

In an embodiment, a value representative of the extent to which the medium in the first filter bed 45 removes temporary hardness is associated with each of several different types of cartridge 43 in the memory 38. Let this value be $\varepsilon$, a value less than 100 % (e.g. e = 0.9). Equations (3)-(6) would be rewritten by dividing by $\varepsilon$, and step 26 in the method of Fig. 2 and step 35 in the method of Fig. 3 adapted accordingly.

In another embodiment, characteristics of the water are inferred from the electrical conductivity measurements and/or the calculated values of the temporary hardness. These characteristics are compared to the type identification of the cartridge. If the cartridge is unsuitable for the characteristics of the water, an appropriate output signal is provided.

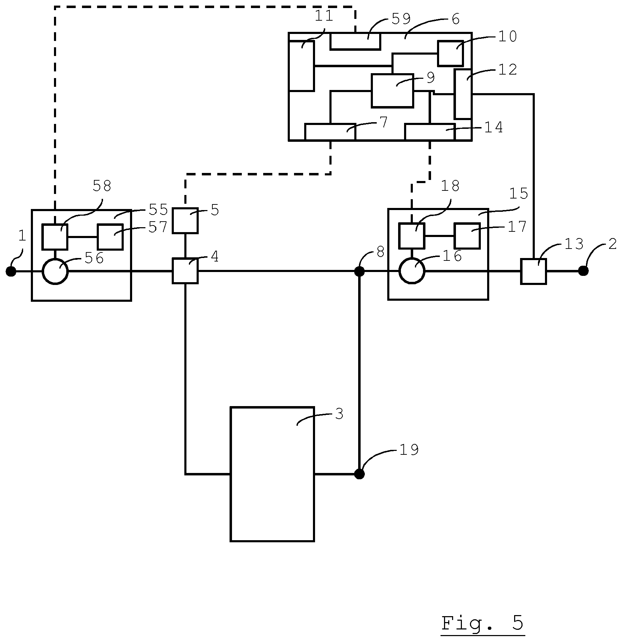

Fig. 5 is a schematic diagram of an alternative water treatment system to the one illustrated in Fig. 1. Like parts have been given like reference numerals. The alternative system differs in that it includes a second sensor device 55. It includes a second electrical conductivity sensor 56 and a second temperature sensor 57, and is arranged to provide measurement values to the control device 6, which is provided with an appropriate further interface 59. The second sensor device 55 includes a data processor 58 adjusting values of the electrical conductivity obtained from the second electrical conductivity sensor 56 to take account of deviations from a reference temperature.

In this variant of the system, the control device 6 only varies the blending fraction x to achieve a target value of the temporary hardness based on a value representative of the temporary hardness of the untreated water. This variant does not, however, require the control device to vary the blending fraction $x$ to obtain measurement values for use in determining the temporary hardness of the untreated water. Instead, the measurement value obtained from the second sensor device 55 is representative of the electrical conductivity $s_0$ of the untreated water (corrected for deviations from a reference temperature).

In the embodiment of Fig. 5, the control device 6 uses a measurement value from the (first) sensor device 15 and a measurement value from the second sensor device 55 to determine the difference $\Delta s \equiv s_0\text{-}s_1$ between the electrical conductivity of the untreated and the treated water. To this end, the control device 6 converts the measurement value $s(x)$ obtained from the (first) sensor device 15 and the measurement value $s_0$ from the second sensor device 55 into the difference value $\Delta s$ as follows:

$$\Delta s = \frac{s_0 - s(x)}{1 - x} \; . \qquad\qquad (7)$$

The temporary hardness $H$ of the untreated water is determined by dividing this value by a factor $F$.

A variant is possible in which the (first) sensor device 15 and second sensor device 55 are calibrated by setting the blending fraction at one (i.e. 100 % bypass) and comparing the measurements from the two devices 15,55 to eliminate systematic errors. Such an operation could be carried out, for example, at the same time as the replacement of the fluid treatment device 3 and with the water from the outlet 2 flushed down the drain to avoid damage to any appliances normally connected to the outlet 2.

It will be noted that methods using the water treatment system illustrated in Fig. 5 make use of a measurement value $s_0$ obtained from a measurement other than one downstream of the mixing location, and that the determination of either the temporary hardness $H$ of the untreated water or the temporary hardness $H^*$ of the water provided at the outlet 2 is determined as a function of the value $x_1$ of the blending fraction prevailing when the measurement with which the measurement value $s(x_1)$ provided by the (first) sensor device 15 was made.

The invention is not limited to the embodiments described above, which may be varied within the scope of the accompanying claims. For instance, it is not absolutely necessary for the second bed 46 not to remove temporary hardness at all. It may be the case that it removes temporary hardness only to a more limited extent than the first bed 45. Through appropriate adaptation of the methods described herein (essentially by multiplying $\Delta s$ by an appropriate factor representative of the difference in effectiveness), the temporary hardness can still be determined, albeit the difference values become much smaller, so that there may be a loss of accuracy. Obviously, the components removable by the fluid treatment part in the first fluid path should not also be completely removable by fluid treatment parts in the second path.

LIST OF REFERENCE NUMERALS

1    - inlet

2    - outlet

3    - fluid treatment device

EP 2 870 472 B1

4      - flow divider

5      - motor

6      - control device

7      - interface to motor

8      - mixing location

9      - data processing unit

10     - memory

11     - interface

12     - interface to flow meter

13     - flow meter

14     - interface to sensor

15     - sensor device

16     - conductivity sensor

17     - temperature sensor

18     - data processor

19     - exit point

20     - step (set blending fraction to reference value)

21     - step (determine conductivity)

22     - step (return to normal operation)

23     - step (set timer)

24     - step (set blending fraction)

25     - step (determine conductivity)

26     - step (calculate temporary hardness)

27     - step (adjust blending fraction)

28     - step (set first blending fraction)

29     - step (obtain first conductivity value)

30     - step (set second blending value)

31     - step (obtain second conductivity value)

32     - step (determine difference value)

33   - step (divide by blending fraction differential)

34   - step (determine conversion factor)

35   - step (convert to temporary hardness)

36   - filter head

37   - data processing unit

38   - memory

39   - inlet connector

40   - outlet connector

41   - flow divider

42   - motor

43   - cartridge

44   - fall tube

45   - first bed

46   - second bed

47   - flow meter

48   - sensor device

49   - inlet connector of sensor device

50   - outlet connector of sensor device

51   - interface to sensor device

52   - token

53   - device for reading data from token

54   - interface

55   - 2nd sensor device

56   - 2nd conductivity sensor

57   - second temperature sensor

58   - data processor

59   - second interface

**Claims**

1.   Method of determining a measure of hardness or temporary hardness, the method including operating a liquid

14

treatment apparatus including:

an inlet (1;39) for untreated water;
a branch point between the inlet (1;39) and at least one first liquid path and at least one second liquid path,
each first liquid path including at least one liquid treatment part (3;45) for treating liquid to remove to at least a certain extent hardness or temporary hardness from water led through the liquid treatment part (3;45),
each second liquid path bypassing at least one of the liquid treatment parts (3;45), the second liquid paths conducting, in use, a blending fraction between zero and one of the water received through the inlet (1;39); and
a mixing location (8;46), where the first and second liquid paths join, the method further including:

obtaining at least two measurement values, each representative of a respective value of a parameter of the water depending partly on the concentration of components removable by the bypassed liquid treatment part (3;45) and partly on the concentration of other components of the water,
wherein the parameter is electrical conductivity or electrical conductivity normalised to a value that would pertain at a reference temperature,
wherein at least a first of the measurement values is a value obtained from a first measurement made downstream of the mixing location (8;46); and
determining an output value representative of the measure for at least one of untreated water and water downstream of the mixing location (8;46) as a function of at least (i) a value representative of the blending fraction prevailing at the first measurement relative to a constant or known reference value of the blending fraction and (ii) a difference between the first measurement value and a second measurement value, the second measurement value being obtained from one of a measurement downstream of the mixing location (8;46) at a different value of the blending fraction than a value of the blending fraction at which the first measurement value is obtained and a measurement carried out on the untreated water,
wherein the measurement to obtain the second measurement value is a measurement other than one downstream of the mixing location (8;46) at a value of the blending fraction of zero.

2. Method according to claim 1,
wherein the second measurement value is obtained from a measurement downstream of the mixing location (8;46) at a different value of the blending fraction than the first.

3. Method according to claim 2,
wherein the liquid treatment apparatus includes at least one device (4,5;41,42) for adjusting the blending fraction, and wherein the first and second measurement values are obtained by causing the device (4,5;41,42) to adjust the blending fraction.

4. Method according to any one of claims 1-3,
wherein the reference value corresponds to a second value of the blending fraction, prevailing at the measurement used to obtain the second measurement value so that the output value is determined as a function of a difference value representative of a change in the blending fraction, and
wherein the step of determining the output value includes dividing the difference between the first and second measurement value by the difference value.

5. Method according to any one of the preceding claims,
wherein the apparatus includes at least one device (4,5;41,42) for adjusting the blending fraction, and
wherein the method includes causing the device (4,5;41,42) to adjust the blending fraction in dependence on the output value.

6. Method according to any one of the preceding claims,
wherein the treatment part (45) is included in a replaceable cartridge (43) for coupling to a device (36) including at least the inlet (39) of the liquid treatment apparatus.

7. Method according to claim 6,
including obtaining input data identifying at least a type of the replaceable cartridge (43), and
at least one of executing and adapting the method in dependence on the identified type.

8. Method according to any one of the preceding claims,
wherein the liquid treatment part comprises a weakly acidic ion exchange medium through which the liquid is led.

9. System for determining a measure of hardness or temporary hardness by operating a liquid treatment apparatus including:

an inlet (1;39) for untreated water;
a branch point between the inlet and at least one first liquid path and at least one second liquid path,
each first liquid path including at least one liquid treatment part (3;45) for treating liquid to remove to at least a certain extent hardness or temporary hardness from water led through the liquid treatment part (3;45),
each second liquid path bypassing at least one of the liquid treatment parts (3;45), the second liquid paths conducting, in use, a blending fraction between zero and one of the water received through the inlet (1;39); and
a mixing location (8;46), where the first and second liquid paths join, the system including:

at least an interface (14;51;59) to at least one sensor (15;48;55) for obtaining at least two measurement values, each representative of a respective value of a parameter of the water depending partly on the concentration of components removable by the bypassed treatment part (3;45) and partly on the concentration of other components of the water,
wherein the parameter is electrical conductivity or electrical conductivity normalised to a value that would pertain at a reference temperature,
wherein the system is configured to obtain at least a first of the measurement values in the form of a value obtained from a first measurement made downstream of the mixing location (8;46); and
a data processing unit (6,9;37) configured to determine an output value representative of the measure for at least one of untreated water and water downstream of the mixing location (8;46) as a function of at least (i) a value representative of the blending fraction prevailing at the first measurement relative to a constant or known reference value of the blending fraction and (ii) a difference between the first measurement value and a second measurement value, the second measurement value being obtained from one of a measurement downstream of the mixing location (8;46) at a different value of the blending fraction than a value of the blending fraction at which the first measurement value is obtained and a measurement carried out on the untreated water,
wherein the system is configured to obtain the second measurement value from a measurement other than one downstream of the mixing location (8;46) at a value of the blending fraction of zero.

10. System according to claim 9,
configured to execute a method according to any one of claims 1-8.

11. System according to any one of claims 9 and 10, adapted for use with a device (36) including at least the inlet (39) of the liquid treatment apparatus and adapted for coupling to a replaceable cartridge (43) including at least the bypassed liquid treatment part (45) of the liquid treatment apparatus,
wherein the system further includes a housing (48) in which at least one of the at least one sensors is arranged, which housing (48) is provided with:

a liquid inlet provided with a coupling device (49) for coupling to an outlet (40) of the device (36) including at least the inlet (39) of the liquid treatment apparatus; and
a liquid outlet provided with a coupling device (50) for coupling to a liquid conduit.

12. System for treating water, including:

an inlet (1;39) for untreated water;
a branch point between the inlet and at least one first liquid path and at least one second liquid path,
each first liquid path including at least one liquid treatment part (3;45) for treating water to remove to at least a certain extent hardness or temporary hardness from water led through the liquid treatment part,
each second liquid path bypassing at least one of the liquid treatment parts (3;45), the second liquid paths conducting, in use, a blending fraction between zero and one of the water received through the inlet;
a mixing location (8;46), where the first and second liquid paths join; and
a system according to any one of claims 9-11.

13. System according to claim 12, wherein the liquid treatment apparatus includes at least one device (4,5;41,42) for adjusting the blending fraction, and
wherein the system includes a component (6,7;37) for causing the device (4,5;41,42) to adjust the blending fraction to obtain the first and second measurement values.

14. Computer program including a set of instructions capable, when incorporated in a machine-readable medium of causing a system having information processing capabilities, a liquid treatment apparatus as defined in claim 1 and an interface for obtaining measurement values representative of respective values of a parameter of water depending partly on the concentration of components removable by the liquid treatment part to carry out a method according to any one of claims 1-8.


**Patentansprüche**

1. Verfahren zur Bestimmung eines Härte- oder temporären Härtemaßes, wobei das Verfahren das Betreiben einer Flüssigkeitsbehandlungseinrichtung umfasst, umfassend:

einen Einlass (1; 39) für unbehandeltes Wasser;
einen Verzweigungspunkt zwischen dem Einlass (1; 39) und mindestens einem ersten Flüssigkeitspfad und mindestens einem zweiten Flüssigkeitspfad,
wobei jeder erste Flüssigkeitspfad mindestens ein Flüssigkeitsbehandlungsteil (3; 45) zum Behandeln von Flüssigkeit umfasst, um Härte oder temporäre Härte aus Wasser, das durch das Flüssigkeitsbehandlungsteil (3; 45) geleitet wird, zumindest bis zu einem gewissen Grad zu beseitigen,
wobei jeder zweite Flüssigkeitspfad mindestens eines der Flüssigkeitsbehandlungsteile (3; 45) umgeht, wobei die zweiten Flüssigkeitspfade im Betrieb einen Verschnittanteil zwischen Null und eins des Wassers, das durch den Einlass (1; 39) erhalten wird, führen; und
eine Mischstelle (8; 46), an dem der erste und der zweite Flüssigkeitspfad sich treffen, wobei das Verfahren ferner beinhaltet:

ein Erhalten von mindestens zwei Messwerten, von denen jeder für einen jeweiligen Wert eines Parameters des Wassers repräsentativ ist, der teilweise von der Konzentration von Komponenten, die durch das umgangene Flüssigkeitsbehandlungsteil (3; 45) entfernt werden können, und teilweise von der Konzentration anderer Komponenten des Wassers abhängt,
wobei der Parameter elektrische Leitfähigkeit oder elektrische Leitfähigkeit normiert auf einen Wert, der bei einer Referenztemperatur gilt, ist,
wobei mindestens ein erster der Messwerte ein Wert ist, der aus einer ersten Messung erhalten wird, die stromabwärts der Mischstelle (8; 46) erfolgt; und
Bestimmen eines Ausgangswertes, der für das Maß für mindestens eines aus unbehandeltem Wasser und Wasser stromabwärts der Mischstelle (8; 46) repräsentativ ist als Funktion von mindestens (i) einem Wert, der für den Verschnittanteil repräsentativ ist, der bei der ersten Messung relativ zu einer Konstanten oder einem bekannten Referenzwert des Verschnittanteils gilt, und (ii) einer Differenz zwischen dem ersten Messwert und einem zweiten Messwert, wobei der zweite Messwert aus einer von einer Messung stromabwärts der Mischstelle (8; 46) bei einem anderen Wert des Verschnittanteils als ein Wert des Verschnittanteils, bei dem der erste Messwert erhalten wird, und einer Messung, die am unbehandelten Wasser durchgeführt wird, erhalten wird,
wobei die Messung, um den zweiten Messwert zu erhalten, eine andere Messung ist als eine Messung stromabwärts der Mischstelle (8; 46) bei einem Wert des Verschnittanteils von Null.

2. Verfahren nach Anspruch 1,
wobei der zweite Messwert aus einer Messung stromabwärts der Mischstelle (8, 46) bei einem anderen Wert des Verschnittanteils als dem ersten erhalten wird.

3. Verfahren nach Anspruch 2,

wobei die Flüssigkeitsbehandlungseinrichtung mindestens eine Vorrichtung (4, 5; 41, 42) zum Anpassen des Verschnittanteils umfasst und
wobei der erste und der zweite Messwert durch Bewirken, dass die Vorrichtung (4, 5; 41, 42) den Verschnittanteil anpasst, erhalten werden.

4. Verfahren nach einem der Ansprüche 1-3,

wobei der Referenzwert einem zweiten Wert des Verschnittanteils entspricht, der bei der Messung, die verwendet wird, um den zweiten Messwert zu erhalten, gilt, sodass der Ausgangswert als Funktion eines Differenzwerts ermittelt wird, der für eine Veränderung des Verschnittanteils repräsentativ ist, und
wobei der Schritt des Bestimmens des Ausgangswerts das Dividieren der Differenz zwischen dem ersten und dem zweiten Messwert durch den Differenzwert umfasst.

5. Verfahren nach einem der vorhergehenden Ansprüche,

   wobei die Einrichtung mindestens eine Vorrichtung (4, 5; 41, 42) zum Anpassen des Verschnittanteils umfasst und
   wobei das Verfahren ein Bewirken, dass die Vorrichtung (4, 5; 41, 42) den Verschnittanteil in Abhängigkeit von dem Ausgangswert anpasst, umfasst.

6. Verfahren nach einem der vorhergehenden Ansprüche,
   wobei das Behandlungsteil (45) in einer auswechselbaren Kartusche (43) zum Koppeln mit einer Vorrichtung (36), die mindestens den Einlass (39) der Flüssigkeitsbehandlungseinrichtung umfasst, umfasst ist.

7. Verfahren nach Anspruch 6,

   welches ein Erhalten von Eingabedaten, die mindestens einen Typ der austauschbaren Kartusche (43) identifizieren und
   mindestens eines aus dem Ausführen und dem Anpassen des Verfahrens in Abhängigkeit von dem identifizierten Typ umfasst.

8. Verfahren nach einem der vorhergehenden Ansprüche,
   wobei das Flüssigkeitsbehandlungteil ein schwach saures Ionenaustauschmedium umfasst, durch welches die Flüssigkeit geleitet wird.

9. System zur Bestimmung eines Härte- oder temporären Härtemaßes durch Betreiben einer Flüssigkeitsbehandlungseinrichtung, die umfassend:

   einen Einlass (1; 39) für unbehandeltes Wasser;
   einen Verzweigungspunkt zwischen dem Einlass und mindestens einem ersten Flüssigkeitspfad und mindestens einem zweiten Flüssigkeitspfad,
   wobei jeder erste Flüssigkeitspfad mindestens ein Flüssigkeitsbehandlungteil (3; 45) zum Behandeln von Flüssigkeit umfasst, um Härte oder temporäre Härte aus Wasser, das durch das Flüssigkeitsbehandlungteil (3; 45) geleitet wird, zumindest bis zu einem gewissen Grad zu beseitigen,
   wobei jeder zweite Flüssigkeitspfad mindestens eines der Flüssigkeitsbehandlungteile (3; 45) umgeht, wobei die zweiten Flüssigkeitspfade bei der Verwendung einen Verschnittanteil zwischen Null und eins des Wassers, das durch den Einlass (1; 39) erhalten wird, führt; und
   eine Mischstelle (8; 46), an dem der erste und der zweite Flüssigkeitspfad sich treffen, wobei das System beinhaltet:

   mindestens eine Schnittstelle (14; 51; 59) mit wenigstens einem Sensor (15; 48; 55) zum Erhalten von mindestens zwei Messwerten, von denen jeder für einen jeweiligen Wert eines Parameters des Wassers repräsentativ ist, der teilweise von der Konzentration von Komponenten, die durch das umgangene Behandlungteil (3; 45) entfernt werden können, und teilweise von der Konzentration anderer Komponenten des Wassers abhängt,
   wobei der Parameter elektrische Leitfähigkeit oder elektrische Leitfähigkeit normiert auf einen Wert, der bei einer Referenztemperatur gilt, ist,
   wobei das System konfiguriert ist, um mindestens einen ersten der Messwerte in Form eines Wertes zu erhalten, der aus einer stromabwärts der Mischstelle (8; 46) vorgenommenen, ersten Messung erhalten wird und
   eine Datenverarbeitungseinheit (6, 9; 37), die konfiguriert ist, um einen Ausgangswert zu bestimmen, der für das Maß für mindestens eines aus unbehandeltem Wasser und Wasser stromabwärts der Mischstelle (8; 46) repräsentativ ist als Funktion von mindestens (i) einem Wert, der für den Verschnittanteil repräsentativ ist, der bei der ersten Messung relativ zu einer Konstanten oder einem bekannten Referenzwert des Mischanteils repräsentativ ist, und (ii) einer Differenz zwischen dem ersten Messwert und einem zweiten Messwert, wobei der zweite Messwert aus einer von einer Messung stromabwärts der Mischstelle (8; 46) bei einem anderen Wert des Verschnittanteils als ein Wert des Mischanteils, bei dem der erste Messwert erhalten wird, und einer Messung, die am unbehandelten Wasser durchgeführt wird, erhalten wird, wobei das System konfiguriert ist, um den zweiten Messwert aus einer anderen Messung als einer Messung stromabwärts der Mischstelle (8, 46) bei einem Wert des Verschnittanteils von Null zu erhalten.

**10.** System nach Anspruch 9,
das konfiguriert ist, um ein Verfahren nach einem der Ansprüche 1-8 auszuführen.

**11.** System nach einem der Ansprüche 9 und 10,

eingerichtet zur Verwendung mit einer Vorrichtung (36), die mindestens den Einlass (39) der Flüssigkeitsbehandlungseinrichtung umfasst und zum Koppeln mit einer austauschbaren Kartusche (43) eingerichtet ist, die mindestens das umgangene Flüssigkeitsbehandlungsteil (45) der Flüssigkeitsbehandlungseinrichtung umfasst, wobei das System ferner ein Gehäuse (48) umfasst, in dem mindestens einer der wenigstens einen Sensoren angeordnet ist, wobei das Gehäuse (48) aufweist:

einen Flüssigkeitseinlass, der eine Kopplungsvorrichtung (49) zum Koppeln mit einem Auslass (40) der mindestens den Einlass (39) der Flüssigkeitsbehandlungseinrichtung umfassenden Vorrichtung aufweist; und
einen Flüssigkeitsauslass, der eine Kopplungsvorrichtung (50) zum Koppeln mit einer Flüssigkeitsleitung aufweist.

**12.** System zum Behandeln von Wasser, umfassend:

einen Einlass (1; 39) für unbehandeltes Wasser;
einen Verzweigungspunkt zwischen dem Einlass und mindestens einem ersten Flüssigkeitspfad und mindestens einem zweiten Flüssigkeitspfad,
wobei jeder erste Flüssigkeitspfad mindestens ein Flüssigkeitsbehandlungsteil (3; 45) zum Behandeln von Wasser umfasst, um Härte oder temporäre Härte aus Wasser, das durch das Flüssigkeitsbehandlungsteil geleitet wird, zumindest bis zu einem gewissen Grad zu entfernen,
wobei jeder zweite Flüssigkeitspfad mindestens eines der Flüssigkeitsbehandlungsteile (3; 45) umgeht, wobei die zweiten Flüssigkeitspfade im Betrieb einen Verschnittanteil zwischen Null und eins des Wassers, das durch den Einlass erhalten wird, führen;
eine Mischstelle (8; 46), an dem der erste und der zweite Flüssigkeitspfad sich treffen; und
ein System nach einem der Ansprüche 9-11.

**13.** System nach Anspruch 12, wobei die Flüssigkeitsbehandlungseinrichtung mindestens eine Vorrichtung (4, 5; 41, 42) zum Anpassen des Verschnittanteils umfasst und
wobei das System eine Komponente (6, 7; 37) umfasst, um zu bewirken, dass die Vorrichtung (4, 5; 41, 42) den Verschnittanteil anpasst, um den ersten und den zweiten Messwert zu erhalten.

**14.** Computerprogramm, das einen Satz von Befehlen umfasst, die, wenn sie in einem maschinenlesbaren Medium integriert sind, bewirken können, dass ein System, das Informationsverarbeitungsfähigkeiten, eine Flüssigkeitsverarbeitungseinrichtung, wie in Anspruch 1 definiert, und eine Schnittstelle zum Erhalten von Messwerten, die repräsentativ sind für jeweilige Werte eines Wasserparameters, der teilweise von der Konzentration von Komponenten abhängt, die durch das Flüssigkeitsbehandlungsteil entfernt werden können, umfasst ein Verfahren nach einem der Ansprüche 1-8 ausführt.

## Revendications

**1.** Procédé pour déterminer une mesure de dureté ou de dureté temporaire, le procédé comprenant faire fonctionner un appareil de traitement de liquide comprenant :

une entrée (1 ; 39) pour eau non traitée ;
un point de ramification entre l'entrée (1 ; 39) et au moins un premier trajet de liquide et au moins un second trajet de liquide,
chaque premier trajet de liquide comprenant au moins une partie de traitement de liquide (3 ; 45) pour traiter du liquide afin d'éliminer, au moins dans une certaine mesure, la dureté ou la dureté temporaire de l'eau acheminée à travers la partie de traitement de liquide (3 ; 45),
chaque second trajet de liquide contournant au moins une des parties de traitement de liquide (3 ; 45), les seconds trajets de liquide conduisant, en utilisation, une fraction de mélange entre zéro et un de l'eau reçue à travers l'entrée (1 ; 39) ; et
un emplacement de mélange (8 ; 46), où les premier et second trajets de liquide se rejoignent, le procédé comprenant en outre :

obtenir au moins deux valeurs de mesure, chacune étant représentative d'une valeur respective d'un paramètre de l'eau dépendant partiellement de la concentration en composants pouvant être éliminés par la partie de traitement de liquide contournée (3 ; 45) et partiellement de la concentration en d'autres composants de l'eau,
le paramètre étant une conductivité électrique ou une conductivité électrique normalisée à une valeur qui se rapporterait à une température de référence,
au moins une première des valeurs de mesure étant une valeur obtenue à partir d'une première mesure réalisée en aval de l'emplacement de mélange (8 ; 46) ; et
déterminer une valeur de sortie représentative de la mesure pour au moins une parmi l'eau non traitée et l'eau en aval de l'emplacement de mélange (8 ; 46) en fonction d'au moins (i) une valeur représentative de la fraction de mélange prévalant à la première mesure par rapport à une valeur de référence constante ou connue de la fraction de mélange et (ii) une différence entre la première valeur de mesure et une seconde valeur de mesure, la seconde valeur de mesure étant obtenue à partir d'au moins une parmi une mesure en aval de l'emplacement de mélange (8 ; 46) à une valeur de fraction de mélange différente d'une valeur de fraction de mélange à laquelle la première valeur de mesure est obtenue, et une mesure réalisée sur l'eau non traitée,
la mesure pour obtenir la seconde valeur de mesure étant une mesure autre que celle en aval de l'emplacement de mélange (8 ; 46) à une valeur de fraction de mélange de zéro.

2.  Procédé selon la revendication 1,
dans lequel la seconde valeur de mesure est obtenue à partir d'une mesure en aval de l'emplacement de mélange (8 ; 46) à une valeur de fraction de mélange différente de la première.

3.  Procédé selon la revendication 2,

dans lequel l'appareil de traitement de liquide comprend au moins un dispositif (4, 5 ; 41, 42) pour ajuster la fraction de mélange, et
les première et seconde valeurs de mesure étant obtenues en amenant le dispositif (4, 5 ; 41, 42) à ajuster la fraction de mélange.

4.  Procédé selon l'une quelconque des revendication 1 à 3,

dans lequel la valeur de référence correspond à une seconde valeur de fraction de mélange, prévalant à la mesure utilisée pour obtenir la seconde valeur de mesure de telle sorte que la valeur de sortie est déterminée en fonction d'une valeur de différence représentative d'un changement de la fraction de mélange, et
dans lequel l'étape de détermination de la valeur de sortie comprend diviser la différence entre les première et seconde valeurs de mesure par la valeur de différence.

5.  Procédé selon l'une quelconque des revendication précédentes,

dans lequel l'appareil comprend au moins un dispositif (4, 5 ; 41, 42) pour ajuster la fraction de mélange, et
le procédé comprend amener le dispositif (4, 5 ; 41, 42) à ajuster la fraction de mélange en fonction de la valeur de sortie.

6.  Procédé selon l'une quelconque des revendication précédentes,
dans lequel la partie de traitement (45) est comprise dans une cartouche remplaçable (43) pour un couplage à un dispositif (36) comprenant au moins l'entrée (39) de l'appareil de traitement de liquide.

7.  Procédé selon la revendication 6,
comprenant obtenir des données d'entrée identifiant au moins un type de la cartouche remplaçable (43), et exécuter et/ou adapter le procédé en fonction du type identifié.

8. Procédé selon l'une quelconque des revendication précédentes,
dans lequel la partie de traitement de liquide comprend un milieu d'échange d'ions faiblement acide à travers lequel le liquide est acheminé.

9. Système pour déterminer une mesure de dureté ou de dureté temporaire en faisant fonctionner un appareil de traitement de liquide comprenant :

une entrée (1 ; 39) pour eau non traitée ;
un point de ramification entre l'entrée et au moins un premier trajet de liquide et au moins un second trajet de liquide,
chaque premier trajet de liquide comprenant au moins une partie de traitement de liquide (3 ; 45) pour traiter du liquide afin d'éliminer, au moins dans une certaine mesure, la dureté ou la dureté temporaire de l'eau acheminée à travers la partie de traitement de liquide (3 ; 45),
chaque second trajet de liquide contournant au moins une des parties de traitement de liquide (3 ; 45), les seconds trajets de liquide conduisant, en utilisation, une fraction de mélange entre zéro et un de l'eau reçue à travers l'entrée (1 ; 39) ; et
un emplacement de mélange (8 ; 46), où les premier et second trajets de liquide se rejoignent, le système comprenant :

au moins une interface (14 ; 51 ; 59) avec au moins un capteur (15 ; 48 ; 55) pour obtenir au moins deux valeurs de mesure, chacune étant représentative d'une valeur respective d'un paramètre de l'eau dépendant partiellement de la concentration en composants pouvant être éliminés par la partie de traitement contournée (3 ; 45) et partiellement de la concentration en d'autres composants de l'eau,
le paramètre étant une conductivité électrique ou une conductivité électrique normalisée à une valeur qui se rapporterait à une température de référence,
le système étant configuré pour obtenir au moins une première des valeurs de mesure sous la forme d'une valeur obtenue à partir d'une première mesure réalisée en aval de l'emplacement de mélange (8 ; 46) ; et
une unité de traitement de données (6, 9 ; 37) configurée pour déterminer une valeur de sortie représentative de la mesure pour au moins une parmi l'eau traitée et l'eau en aval de l'emplacement de mélange (8 ; 46) en fonction d'au moins (i) une valeur représentative de la fraction de mélange prévalant à la première mesure par rapport à une valeur de référence constante ou connue de la fraction de mélange et (ii) une différence entre la première valeur de mesure et une seconde valeur de mesure, la seconde valeur de mesure étant obtenue à partir d'au moins une parmi une mesure en aval de l'emplacement de mélange (8 ; 46) à une valeur de fraction de mélange différente d'une valeur de fraction de mélange à laquelle la première valeur de mesure est obtenue, et une mesure réalisée sur l'eau non traitée,
le système étant configuré pour obtenir la seconde valeur de mesure à partir d'une mesure autre que celle en aval de l'emplacement de mélange (8 ; 46) à une valeur de fraction de mélange de zéro.

10. Système selon la revendication 9,
configuré pour exécuter un procédé selon l'une quelconque des revendications 1 à 8.

11. Système selon l'une quelconque des revendications 9 et 10,

adapté pour être utilisé avec un dispositif (36) comprenant au moins l'entrée (39) de l'appareil de traitement de liquide et adapté pour être couplé à une cartouche remplaçable (43) comprenant au moins la partie de traitement de liquide contournée (45) de l'appareil de traitement de liquide,
le système comprenant en outre un boîtier (48) dans lequel au moins un parmi l'au moins un capteur est disposé, lequel boîtier (48) comprenant :

une entrée de liquide ayant un dispositif de couplage (49) pour un couplage à une sortie (40) du dispositif (36) comprenant au moins l'entrée (39) de l'appareil de traitement de liquide ; et
une sortie de liquide ayant un dispositif de couplage (50) pour un couplage à une conduite de liquide.

12. Système de traitement d'eau, comprenant :

une entrée (1 ; 39) pour eau non traitée ;
un point de ramification entre l'entrée et au moins un premier trajet de liquide et au moins un second trajet de liquide,

chaque premier trajet de liquide comprenant au moins une partie de traitement de liquide (3 ; 45) pour traiter l'eau afin d'éliminer, au moins dans une certaine mesure, la dureté ou la dureté temporaire de l'eau acheminée à travers la partie de traitement de liquide,

chaque second trajet de liquide contournant au moins une des parties de traitement de liquide (3 ; 45), les seconds trajets de liquide conduisant, en utilisation, une fraction de mélange entre zéro et un de l'eau reçue à travers l'entrée ;

un emplacement de mélange (8 ; 46), où les premier et second trajets de liquide se rejoignent ; et

un système selon l'une quelconque des revendications 9 à 11.

13. Système selon la revendication 12, dans lequel l'appareil de traitement de liquide comprend au moins un dispositif (4, 5 ; 41, 42) pour ajuster la fraction de mélange, et

le système comprenant un composant (6, 7 ; 37) pour amener le dispositif (4, 5 ; 41, 42) à ajuster la fraction de mélange pour obtenir les première et seconde valeurs de mesure.

14. Programme d'ordinateur comprenant un ensemble d'instructions capables, lorsqu'il est incorporé dans un support lisible par machine, d'amener un système ayant des capacités de traitement d'informations, un appareil de traitement de liquide tel que défini dans la revendication 1 et une interface pour obtenir des valeurs de mesure représentatives de valeurs respectives d'un paramètre de l'eau dépendant partiellement de la concentration en composants pouvant être éliminés par la partie de traitement de liquide à réaliser un procédé selon l'une quelconque des revendications 1 à 8.

Fig. 1

Fig. 2

Fig. 3

Fig. 4

Fig. 5

**REFERENCES CITED IN THE DESCRIPTION**

**Patent documents cited in the description**

- EP 2169392 A1
- DE 102010061179 A1